# EUROPEAN PATENT SPECIFICATION

(11) **EP 2 349 254 B1**
(45) Date of publication and mention of the grant of the patent: **25.07.2018**
(21) Application number: 09827701.5
(22) Date of filing: 17.11.2009
(51) Int. Cl.: A61K 31/366, A61K 31/352, A61P 17/00, A61K 9/00

(54) **SKIN EXTERNAL COMPOSITION FOR INHIBITING EPIDERMAL HYPERPROLIFERATION AND ALLEVIATING INFLAMMATORY SKIN DISEASES CONTAINING ORTHO-DIHYDROXYISOFLAVONE DERIVATIVES**
TOPISCHE HAUTZUSAMMENSETZUNG ZUR UNTERDRÜCKUNG DER EPIDERMALEN HYPERPROLIFERATION UND LINDERUNG VON ENTZÜNDLICHEN HAUTERKRANKUNGEN MIT ORTHO-DIHYDROXYISOFLAVON-DERIVATEN
COMPOSITION EXTERNE POUR LA PEAU CONTENANT DES DÉRIVÉS D'ORTHO-DIHYDROXYISOFLAVONE ET POUVANT EMPÊCHER L'HYPERPROLIFÉRATION ÉPIDERMIQUE ET ATTÉNUER LES AFFECTIONS CUTANÉES INFLAMMATOIRES

(30) Priority: 18.11.2008 KR 20080114594
(43) Date of publication of application: 03.08.2011
(73) Proprietor: Amorepacific Corporation, Seoul 140-777 (KR)
(72) Inventor: PARK, Jun Seong, Suwon-si Gyeonggi-do 443-738 (KR); PARK, Hye Yoon, Anyang-si Gyeonggi-do 431-713 (KR); KIM, Dong Hyun, Uiwang-si Gyeonggi-do 437-040 (KR); LEE, Sang Min, Yongin-si Gyeonggi-do 446-729 (KR); SHIN, Dong Wook, Seoul 120-070 (KR); KIM, Shin Hyoung, Yongin-si Gyeonggi-do 446-763 (KR); NOH, Min Soo, Yongin-si Gyeonggi-do 446-729 (KR); KIM, Duck Hee, Seoul 137-030 (KR); KIM, Han Kon, Suwon-si Gyeonggi-do 443-793 (KR)
(74) Representative: Patent- und Rechtsanwälte Ullrich & Naumann
(86) International application number: PCT/KR2009/006744
(87) International publication number: WO 2010/058936

(56) References cited:
- WO-A1-2009/131264
- KR-A- 20080 088 799
- SERAFIM KIRIAKIDIS ET AL.: 'Novel tempeh(fermented soyabean) isoflavones inhibit in vivo angiogenesis in the chichen chorioallantoic membrane assay.' BRITISH JOURNAL OF NUTRITION. vol. 93, no. 3, March 2005, pages 317 - 323, XP008145925
- TE-SHENG CHANG ET AL.: 'Identifying 6,7,4'-Trihydroxyisoflavone as a Potent Tyrosinase Inhibitor.' BIOSCIENCE BIOTECHNOLOGY AND BIOCHEMISTRY. vol. 69, no. 10, October 2005, pages 1999 - 2001, XP008145927
- SABINE E. KULLING ET AL.: 'Oxidative Metabolism of the Soy Isoflavones Daidzein and Genistein in Humans in Vitro and in Vivo.' JOURNAL OF AGRICULTURAL AND FOOD CHEMISTRY vol. 49, no. 6, June 2001, pages 3024 - 3033, XP008145919

## Description

### Technical Field

The present invention relates to a skin external composition for inhibiting epidermal hyperproliferation and alleviating inflammatory skin diseases, which contains ortho-dihydroxyisoflavone derivatives as an active ingredient, and more particularly to a skin external composition for inhibiting epidermal hyperproliferation and alleviating inflammatory skin diseases, which contains 4',6,7-trihydroxyisoflavone, 3',4',7-trihydroxyisoflavone or a mixture thereof, which are ortho-dihydroxyisoflavone derivatives, and thus increases the expression of DKK3 protein and FZD1 protein, which appropriately control Wnt signaling, thereby inhibiting epidermal hyperproliferation and alleviating inflammatory skin diseases caused by epidermal hyperproliferation.

### Background Art

Keratinocytes maintain skin moisture levels through their proliferation and differentiation and function as a skin barrier. However, if the proliferation and differentiation of keratinocytes are abnormal, the skin barrier will be impaired, and various skin diseases will occur.

For example, psoriasis is an inflammatory skin disease caused by the abnormal proliferation and differentiation of keratinocytes. It is known that one of the causes of psoriasis is abnormality of the protein beta-catenin. Beta-catenin generally has two functions: to contribute cell-cell adhesion; and to mediate Wnt signaling. Wnt ligands are expressed in normal skin and are generally used in the regulation of proliferation of keratinocytes over several generations during the growth and differentiation of skin cells. However, the Wnt ligands are excessively expressed or absent, the regulation of skin homeostasis will be seriously affected.

In clinical experiments conducted using skin cells isolated from normal persons and psoriasis patients, it was observed that the expression of the Wnt signaling mediator beta-catenin was significantly high in the psoriasis patients and that enzymes which are regulated by beta-catenin are also significantly influenced. Accordingly, if abnormal skin differentiation occurs, it can be predicted that the Wnt signaling system may be one of the causes of the abnormal skin differentiation and that the regulation of the Wnt signaling system will contribute to the protection of the skin barrier and the maintenance of skin moisture. If a method of enhancing the expression of the DKK3 (Dickkopf homolog 3) and FZD1 (Frizzled homolog 1) known to inhibit Wnt signaling is considered, it is predicted that the method will function to suitably control the function of Wnt signaling, thus maintaining skin moisture and protecting the skin barrier.

### Disclosure of Invention

### Technical Problem

The present inventors have conducted studies to find materials for regulating the Wnt signaling that causes the hyperproliferation and differentiation of keratinocytes, resulting in inflammatory skin diseases, and, as a result, have found that the ortho-dihydroxyisoflavone derivatives, 4',6,7-trihydroxyisoflavone or 3',4',7-trihydroxyisoflavone, have an excellent effect of regulating Wnt signaling by increasing the expression of the Wnt signaling inhibitors DKK3 protein and FZD1 protein, thereby completing the present invention.

It is therefore an object of the present invention to provide a skin external composition which increases the expression of DKK3 protein and FZD1 protein, which regulate Wnt signaling, thereby inhibiting epidermal hyperproliferation and alleviating inflammatory skin diseases caused by epidermal hyperproliferation.

### Solution to Problem

To achieve the above object, the present invention provides a skin external composition for inhibiting epidermal hyperproliferation and alleviating inflammatory skin diseases, which contains, as an active ingredient, 4',6,7-trihydroxyisoflavone, 3',4',7-trihydroxyisoflavone or a mixture thereof, which are ortho-dihydroxyisoflavone derivatives.

The present invention also provides an anti-inflammatory therapeutic agent containing, as an active ingredient, 4',6,7-trihydroxyisoflavone, 3',4',7-trihydroxyisoflavone or a mixture thereof, which are ortho-dihydroxyisoflavone derivatives.

### Advantageous Effects of Invention

The skin external composition according to the present invention contains ortho-dihydroxyisoflavone derivatives as an active ingredient, and thus increases the expression of DKK3 protein and FZD1 protein, which appropriately regulate Wnt signaling that causes the hyperproliferation of keratinocytes, thereby inhibiting the hyperproliferation of keratinocytes and inducing the normal differentiation of keratinocytes.

In addition, the skin external composition according to the present invention has an excellent effect of alleviating and treating inflammatory skin diseases caused by epidermal hyperproliferation, and thus has an excellent effect of improving skin conditions.

### Brief Description of Drawings

FIG. 1 shows the results obtained by measuring the ketatinocyte division-inhibiting effects of ortho-dihydroxyisoflavone derivatives of the present invention.
FIG. 2 shows the results obtained by measuring the change in expression of DKK3 caused by ortho-dihydroxyisoflavone derivatives of the present invention.
FIG. 3 shows the results obtained by measuring the change in expression of FZD 1 caused by ortho-dihydroxyisoflavone derivatives of the present invention.
FIG. 4 shows the results obtained by measuring the anti-inflammatory effects of ortho-dihydroxyisoflavone derivatives of the present invention.

### Best Mode for Carrying out the Invention

Hereinafter, the present invention will be described in detail.

The present invention provides a skin external composition for inhibiting epidermal hyperproliferation and treating inflammatory skin diseases, the composition containing, as an active ingredient, 4',6,7-trihydroxyisoflavone, 3',4',7-trihydroxyisoflavone or a mixture thereof, which are ortho-dihydroxyisoflavone (ODI) derivatives.

The present invention also provides an anti-inflammatory therapeutic agent containing, as an active ingredient, 4',6,7-trihydroxyisoflavone, 3',4',7-trihydroxyisoflavone or a mixture thereof, which are ortho-dihydroxyisoflavone derivatives.

The ortho-dihydroxyisoflavone derivatives have an antioxidant effect higher than those of other isoflavones. Isoflavones are vegetable compounds which are contained mainly in beans. Isoflavones are present in the form of glycosides, which contain isoflavones as aglycons and are converted into aglycon forms during fermentation by microbial metabolism.

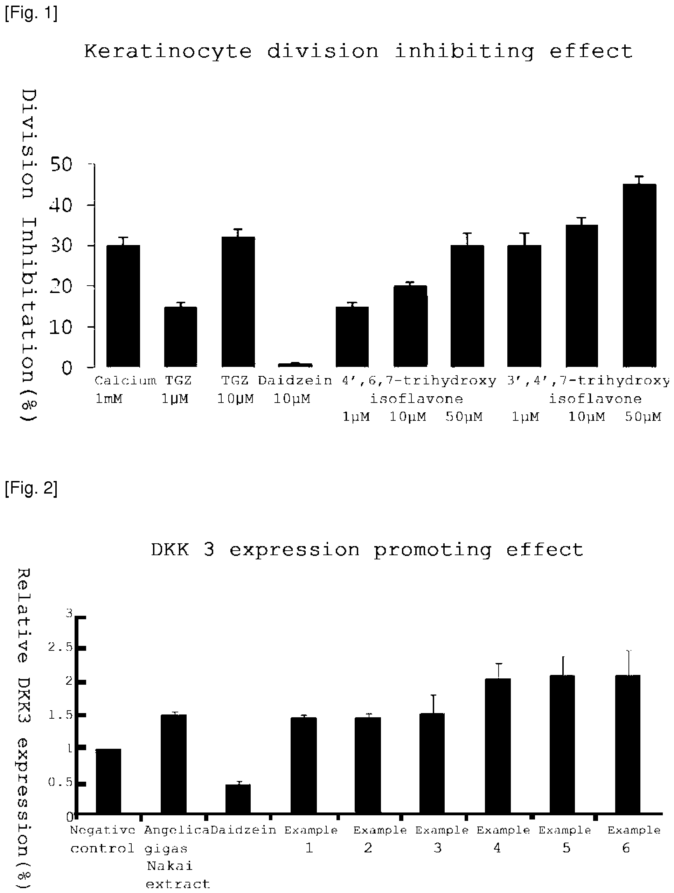

The ortho-dihydroxyisoflavone derivatives which are used as an active ingredient in the present invention are 4',6,7-trihydroxyisoflavone (represented by the following Chemical Figure 1) or 3',4',7-trihydroxyisoflavone (represented by the following Chemical Figure 2). These derivatives show the effect of effectively increasing the expression of the Wnt signaling inhibitors DKK3 (Dickkopf homolog 3) and FZD 1 (Frizzled homolog 1) to inhibit the hyperproliferation of keratinocytes, thereby inducing the normal differentiation of keratinocytes and alleviating and treating inflammatory skin diseases caused by the hyperproliferation of keratinocytes.

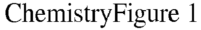

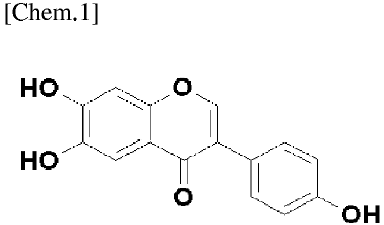

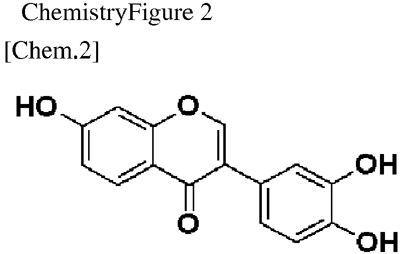

4',6,7-trihydroxyisoflavone and 3',4',7-trihydroxyisoflavone, which are the ortho-dihydroxyisoflavone derivatives of the present invention, can be prepared by bio-transforming daidzein according to a method well known in the art, but the preparation method is not limited only thereto and may be any conventional method known in the art.

The ortho-dihydroxyisoflavone derivatives are preferably contained in an amount of 0.001-30 wt% based on the total weight of the composition. If the content of the derivatives is less than 0.001 wt%, the effect of inhibiting epidermal hyperproliferation and ameliorating inflammatory skin diseases cannot be obtained, and if the content exceeds 30 wt%, it will be inefficient because the effect thereof will not be greatly increased, and furthermore, a problem associated with formulation stability will occur.

The skin external composition according to the present invention can be prepared in the form of a pharmaceutical composition containing an effective amount of 4',6,7-trihydroxyisoflavone, 3',4',7-trihydroxyisoflavone or a mixture thereof and may contain one or more nontoxic, pharmaceutically acceptable carriers, adjuvants, diluents or other active ingredients, which are conventionally used in the art. The skin external composition according to the present invention can be formulated using pharmaceutically acceptable carriers and vehicles according to a known method.

Specifically, the composition of the present invention may be used in the form of suitable pharmaceutical formulations, including external formulations such as ointment, gel, cream, patch and spray. These formulations may contain various suitable bases and additives required for the preparation thereof, and the types and amount of these bases and additives can be easily selected by a person skilled in the art.

Moreover, the skin external composition according to the present invention may be formulated in the form of a solution, a suspension or an emulsion in oil or aqueous medium or may be formulated in the form of dry powder which is dissolved in sterile pyrogen-free water before use. The skin external composition of the present invention can also be in the form of water-in-oil emulsions. The oily phase can be a vegetable oil such as olive oil, or a mineral oil such as liquid paraffin, and the emulsifying agent can be: naturally occurring phospholipids such as soybean-lecithin; esters or partial esters derived from fatty acids and hexitol anhydrides such as sorbitan monooleate; or condensation products of said partial esters with ethylene oxide such as polyoxyethylene sorbitan monooleate.

The skin external composition of the present invention can be formulated as cosmetic preparations. Examples of the cosmetic preparations include, but are not limited to, skin softener, astringent lotion, milk lotion, eye cream, nourishing cream, massage cream, cleansing cream, cleansing foam, cleansing water, powder, essence and pack.

### Mode for the Invention

Hereinafter, the present invention will be described in further detail with reference to preparation examples, examples and test examples. It is to be understood, however, that these examples are for illustrative purposes only and are not to be construed to limit the scope of the present invention. Also, those skilled in the art will appreciate that various modifications, additions and substitutions are possible, without departing from the scope and spirit of the present invention as disclosed in the accompanying claims.

### Preparation Example 1: Preparation of soybean extract

2 kg of soybean was added to 6 ℓ of hexane and extracted three times with stirring at room temperature so as to be defatted. 1 kg of the defatted soybean was added to 4 ℓ of 80% methanol, extracted three times under reflux and dipped at 15°C for 1 day. The extract was filtered through filter cloth and centrifuged to separate it into the residue and the filtrate, and the separated filtrate was concentrated under reduced pressure. The concentrate was suspended in water, and then extracted five times with 1 ℓ of ether to remove the pigment, and the aqueous layer was extracted three times with 500 mℓ of 1-butanol. The resulting 1-butanol layer was concentrated under reduced pressure to obtain a 1-butanol extract. The extract was dissolved in a small amount of methanol, and then a large amount of ethyl acetate was added thereto. The produced precipitate was dried, thus obtaining 300 g of a soybean extract.

### Example 1: Preparation of 4',6,7-trihydroxyisoflavone using soybean extract

10 g of the soybean extract obtained in Preparation Example 1 was dissolved in 100 mℓ of ionized water, sterilized at 121 °C for 30 minutes and cooled to 30 °C. Then, previously cultured *Aspergillus niger* KCCM 11885 was inoculated into the liquid in an amount of 5-10 wt% based on the weight of the liquid. The inoculated microorganism was cultured at 37 °C for 7 days. The depletion rate of the substrate was analyzed by thin layer chromatography, and when the substrate was completely depleted, the culture was terminated. The culture broth was centrifuged at 5,000-10,000 rpm to obtain a precipitate. The precipitate was washed three times with distilled water and centrifuged again to obtain a precipitate. The precipitate was added to 200 mℓ of ethanol, stirred three times, and filtered to remove the precipitated salts. The filtrate was concentrated under reduced pressure to obtain a crude product. The obtained crude product was purified by silica gel column chromatography (chloroform: methanol = 8:1-4:1), thus obtaining 0.23 g of 4',6,7-trihydroxyisoflavone.

### Example 2: Preparation of 4',6,7-trihydroxyisoflavone using daidzin

5 g of daidzin (Sigma) was dissolved in 100 mℓ of ionized water, sterilized at 121 °C for 30 minutes and cooled to 30 °C. Then, previously cultured *Aspergillus niger* KCCM 11885 was inoculated into the liquid in an amount of 5-10 wt% based on the weight of the liquid. The inoculated microorganism was cultured at 37 °C for 7 days. The depletion rate of the substrate was analyzed by thin layer chromatography, and when the substrate was completely depleted, the culture was terminated. The culture broth was centrifuged at 5,000-10,000 rpm to obtain a precipitate. The precipitate was washed three times with distilled water and centrifuged again to obtain a precipitate. The precipitate was added to 200 mℓ of ethanol, stirred three times, and filtered to remove the precipitated salts. The filtrate was concentrated under reduced pressure to obtain a crude product. The obtained crude product was purified by silica gel column chromatography (chloroform: methanol = 8:1-4:1), thus obtaining 0.34 g of 4',6,7-trihydroxyisoflavone.

### Example 3: Preparation of 4',6,7-trihydroxyisoflavone using daidzein

3 g of daidzein (Sigma) was dissolved in 100 mℓ of ionized water, sterilized at 121 °C for 30 minutes and cooled to 30 °C. Then, previously cultured *Aspergillus niger* KCCM 11885 was inoculated into the liquid in an amount of 5-10 wt% based on the weight of the liquid. The inoculated microorganism was cultured at 37 °C for 7 days, after which the culture was terminated. The culture broth was centrifuged at 5,000-10,000 rpm to obtain a precipitate. The precipitate was washed three times with distilled water and centrifuged again to obtain a precipitate. The precipitate was added to 200 mℓ of ethanol, stirred three times, and filtered to remove the precipitated salts. The filtrate was concentrated under reduced pressure to obtain a crude product. The obtained crude product was purified by silica gel column chromatography (chloroform: methanol = 8:1-4:1), thus obtaining 0.45 g of 4',6,7-trihydroxyisoflavone.

### Example 4: Preparation of 3',4',7-trihydroxyisoflavone using soybean extract

10 g of the soybean extract obtained in Preparation Example 1 was dissolved in 100 mℓ of ionized water, sterilized at 121 °C for 30 minutes and cooled to 30 °C. Then, previously cultured *Bacillus subtilis* KCCM 11732 was inoculated into the liquid in an amount of 5-10 wt% based on the weight of the liquid. The inoculated microorganism was cultured at 37 °C for 7 days. The depletion rate of the substrate was analyzed by thin layer chromatography, and when the substrate was completely depleted, the culture was terminated. The culture broth was centrifuged at 5,000-10,000 rpm to obtain a precipitate. The precipitate was washed three times with distilled water and centrifuged again to obtain a precipitate. The precipitate was added to 200 mℓ of ethanol, stirred three times, and filtered to remove the precipitated salts. The filtrate was concentrated under reduced pressure to obtain a crude product. The obtained crude product was purified by silica gel column chromatography (chloroform: methanol = 8:1-4:1), thus obtaining 0.23 g of 3',4',7-trihydroxyisoflavone.

### Example 5: Preparation of 3',4',7-trihydroxyisoflavone using daidzin

5 g of daidzin (Sigma) was dissolved in 100 mℓ of ionized water, sterilized at 121 °C for 30 minutes and cooled to 30 °C. Then, previously cultured *Bacillus subtilis* KCCM 11732 was inoculated into the liquid in an amount of 5-10 wt% based on the weight of the liquid. The inoculated microorganism was cultured at 37 °C for 7 days. The depletion rate of the substrate was analyzed by thin layer chromatography, and when the substrate was completely depleted, the culture was terminated. The culture broth was centrifuged at 5,000-10,000 rpm to obtain a precipitate. The precipitate was washed three times with distilled water and centrifuged again to obtain a precipitate. The precipitate was added to 200 mℓ of ethanol, stirred three times, and filtered to remove the precipitated salts. The filtrate was concentrated under reduced pressure to obtain a crude product. The obtained crude product was purified by silica gel column chromatography (chloroform: methanol = 8:1-4:1), thus obtaining 0.44 g of 3',4',7-trihydroxyisoflavone.

### Example 6: Preparation of 3',4',7-trihydroxyisoflavone using daidzein

3 g of daidzein (Sigma) was dissolved in 100 mℓ of ionized water, sterilized at 121 °C for 30 minutes and cooled to 30 °C. Then, previously cultured *Bacillus subtilis* KCCM 11732 was inoculated into the liquid in an amount of 5-10 wt% based on the weight of the liquid. The inoculated microorganism was cultured at 37 °C for 7 days, after which the culture was terminated. The culture broth was centrifuged at 5,000-10,000 rpm to obtain a precipitate. The precipitate was washed three times with distilled water and centrifuged again to obtain a precipitate. The precipitate was added to 200 mℓ of ethanol, stirred three times, and filtered to remove the precipitated salts. The filtrate was concentrated under reduced pressure to obtain a crude product. The obtained crude product was purified by silica gel column chromatography (chloroform: methanol = 8:1-4:1), thus obtaining 0.45 g of 3',4',7-trihydroxyisoflavone.

### Test Example 1: Keratinocyte division-inhibiting effects of ortho-dihydroxyisoflavone derivatives

Human keratinocyte HaCaT cells were added to 10% fetal bovine serum-containing DMEM medium [Dulbecco's modified Eagle's Medium; Gibco 1210-0038] and cultured in a 24-well plate at a confluence of 70%. The HaCaT cells were treated with 1, 10 and 50 µM of each of 4',6,7-trihydroxyisoflavone and 3',4',7-trihydroxyisoflavone, and after 48 hours, subjected to BrdU analysis. At 12 hours before the BrdU analysis was carried out, BrdU labeling was carried out. The BrdU analysis was carried out using a BrdU colorimetric ELISA kit (Roche) according to the manufacturer's instruction. First, the culture broth was treated with BrdU solution for 12 hours, and then the medium was removed. The cells were fixed, and a peroxidase-conjugated BrdU antibody was added thereto, after which the cells were treated with a substrate solution to induce color development. The degree of color development was measured with an ELISA reader. As positive controls, calcium known to be used for the differentiation of keratinocytes, and troglitazone (TGZ) known to inhibit cell division and induce cell differentiation were used.

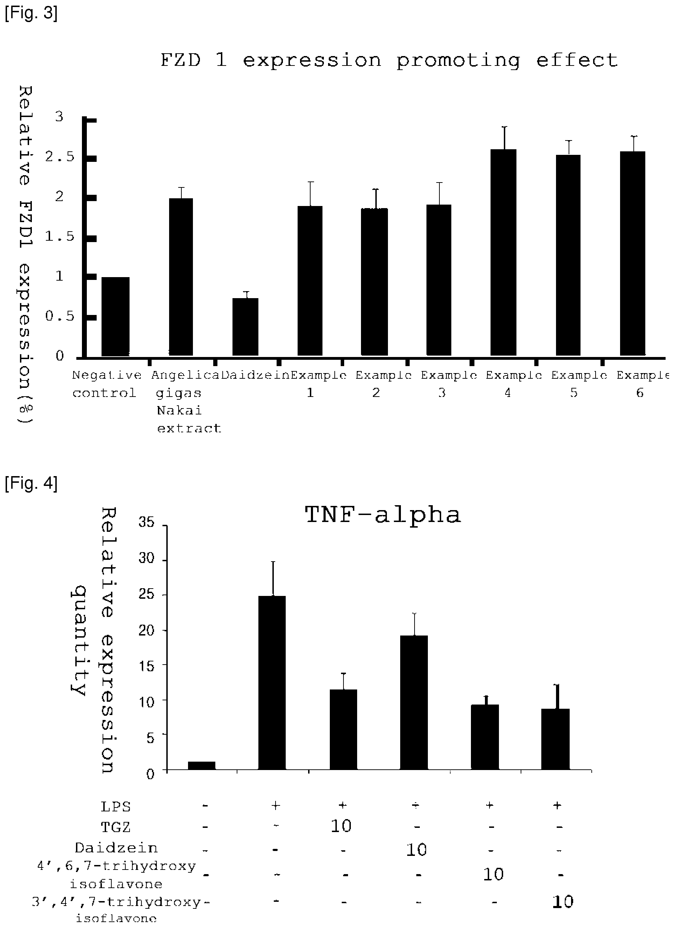

As can be seen in FIG. 1,4',6,7-trihydroxyisoflavone and 3',4',7-trihydroxyisoflavone of the present invention showed keratinocyte division-inhibiting effects which were significantly superior to that of daidzein that is a kind of isoflavone. When the compounds of the present invention were used at a concentration of 50 µM to treat the cells, they showed keratinocyte division-inhibiting effects which were similar or superior to those of the positive controls calcium and troglitazone. Also, 4',6,7-trihydroxyisoflavone and 3',4',7-trihydroxyisoflavone of the present invention showed the effect of inhibiting keratinocyte division in a concentration-dependent manner.

### Test Example 2: DKK3 and FZD1 expression-promoting effect of ortho-dihydroxyisoflavone derivatives

Human neonatal keratinocyte cells purchased from Welskin Co (Seoul, Korea) were subcultured in a 25 cm² T-flask and incubated in a CO₂ incubator under conditions of 37 °C and 5% CO₂. Generally, the experiment was carried out after the cells have been subcultured for 2-3 passages. According to the method of Lonza, Inc. (Walkersville, MD, USA), the medium used in the cell culture was prepared by adding a KGM-2 bullet kit (bovine pituitary extract (2mℓ), human epidermal growth factor (0.5mℓ), insulin (0.5m#), hydrocortisone (0.5m#), transferrin (0.5m#), epinephrine (0.5m#), and gentamicin sulfate + amphotericin-B (GA-1000, 0.5mℓ)) to 500 mℓ of KBM-2 medium (Clonetics CC-3103). When a confluence of about 50% was shown 2 days after the subculture of the cells, the serum was starved. After 24 hours, the cells were treated with 10 µM of each of a negative control, an *Angelica gigas Nakai* extract (positive control), daidzein and Examples 1-6 for 24 hours. As the negative control, an untreated group was used, and the positive control *Angelica gigas Nakai* extract used was obtained by extracting 5 kg of dried *Angelica gigas Nakai* in 50 ℓ of methanol for 24 hours, filtering the extract and concentrating the filtrate under reduced pressure.

Then, RNA of the cells was isolated using Trizol reagent (Invitrogen, Carlsbad, CA, USA). The isolated RNA was purified with Qiagen RNeasy kit (Qiagen, Valencia, CA), and then the quality of the RNA was confirmed using the Agilent 2100 Bio-Analyzer (Agilent Technologies, Santa Clara, CA, USA). cDNA was synthesized from the RNA using a superscript reverse transcriptase (RT) kit (Invitrogen, Carlsbad, CA) and was quantitatively analyzed through a real time-reverse transcription polymerase chain reaction (Q-RT-PCR). The changes in the expression patterns of genes in the keratinocyte cells were analyzed using the TaqMan®gene expression assay kit (AppliedBiosystems, FosterCity, CA), Dickkopf homolog 3 (DKK3)-HS00247426_m1, and Frizzled homolog 1(FZD1)-HS00268943_s1 (TagMan primer catalog name), thus confirming the expression levels of DKK3 and FZD1. The analysis results are shown in FIGS. 2 and 3.

As can be seen in FIGS. 2 and 3, in the case where the cells were treated with the ortho-dihydroxyisoflavone derivatives of the present invention, the expression levels of DKK3 and FZD1 were increased compared to the cases of the negative control and the dihydroxyisoflavone daidzein. Also, the expression levels of DKK3 and FZD1 in the group treated with daidzein that is a kind of isoflavone were significantly lower than those in the groups treated with the ortho-dihydroxyisoflavone derivatives of the present invention. Accordingly, it can be seen that 4',6,7-trihydroxyisoflavone or 3',4',7-trihydroxyisoflavone of the present invention effectively promoted the expression of DKK3 and FZD1.

### Test Example 3: anti-inflammatory effects of ortho-dihydroxyisoflavone derivatives

In this Example, human keratinocyte HaCaT cells obtained from the Korean Cell Line Bank (Seoul, Korea) were used. The HaCaT cells were seeded into a DMEM medium containing 10% (v/v) FBS, 100 U/mℓ penicillin and 100 µg/mℓ streptomycin and were incubated in an animal cell incubator under conditions of 37 °C and 5% CO₂. The HaCaT cells prepared at 1.5 x 10⁶ cells/well were adapted in FBS-free medium for 3hours.

The cells were pretreated with 10 µM of each of 4',6,7-trihydroxyisoflavone, 3',4',7-trihydroxyisoflavone, daidzein and TGZ for 2 hours, 1 *µ*g/mℓ of lipopolysaccharide (LPS) was added thereto, and then the cells were additionally cultured for 8 hours. Total RNA of the cells was extracted using TRIzolTM (GIBCO BRL, MD, USA) and stored at -80 °C.

1 *µ*g of the total RNA was added to 25 *µ*ℓ of a reverse transcription reaction buffer containing 50 mM Tris-HCl (pH 8.3), 75 mM KCl, 3 mM MgCl₂, 0.1 M DTT, 10 mM dNTP and 40 U/*µ*ℓ RNase inhibitor. 0.5 *µ*g/*µ*ℓ oligo(dT)₁₆ primers and 200 U SuperScript II reverse transcriptase (GiboBRL) were added thereto, and the total RNA was subjected to a reverse transcription reaction at 42 °C for 1 hour. Then, 2.5 *µ*ℓ of the reverse transcription reaction solution was added to 50 liters of a PCR reaction buffer containing 0.04 U AmpliTaq DNA polymerase (Perkin Elmer, Shelton, CT), 50 mM Tris-HCl (pH 8.3), 0.25 mg/mℓ bovine serum albumin, 3 mM MgCl₂, 0.25 mM dNTPs and a 1/50,000 dilution of SYBR green I (Molecular Probes, Eugene, OR), and 10 µM primers were added thereto. Then, a PCR reaction was subjected to PCR amplification. The PCR reaction consisted of 30 cycles of denaturation at 94 °C for 30 sec, annealing at 53 °C for 30 sec and extension at 72 °C for 1 min.

The relative mRNA levels were analyzed by measuring the change in fluorescence of SYBR green I using ICycler software. As an internal standard, GAPDH (glyceraldehyde 3-phosphate dehydrogenase) was used to normalize the quantitative expression level of the gene TNF-alpha. The analysis results are shown in FIG. 4.

TNF-alpha which is an inflammation-related factor playing an important in the inflammatory phase of the wound healing process have been frequently used to confirm inflammation inhibitory effects in many experimental papers. Also, it is a gene which is expressed in lipopolysaccharide (LPS)-induced inflammatory reactions in various ways.

As can be seen from the results in FIG. 4, in the group treated with 4',6,7-trihydroxyisoflavone or 3',4',7-trihydroxyisoflavone of the present invention, the expression of the TNF-alpha gene was strongly inhibited. Also, in the group treated with daidzein that is a kind of isoflavone, the expression of the inflammation-related gene was slightly inhibited, but the TNF-alpha expression-inhibiting effect of daidzein was significantly lower than those of the ortho-dihydroxyisoflavone derivatives of the present invention.

As described above, 4',6,7-trihydroxyisoflavone or 3',4',7-trihydroxyisoflavone which is used as an active ingredient in the present invention increases the expression of DKK3 protein and FZD protein, which effectively inhibit keratinocyte division and appropriately control Wnt signaling. Thus, 4',6,7-trihydroxyisoflavone or 3',4',7-trihydroxyisoflavone of the present invention has the effects of inhibiting epidermal hyperproliferation and alleviating inflammatory diseases caused by epidermal hyperproliferation. Therefore, the present invention provides an agent for inhibiting epidermal hyperproliferation or an agent for alleviating inflammatory skin diseases, the agents containing 4',6,7-trihydroxyisoflavone or 3',4',7-trihydroxyisoflavone as an active ingredient.

### Formulation Example 1: Milk lotion

According to the composition shown in Table 1 below, a milk lotion was prepared using a conventional method.

### Table 1

### [Table 1]

**[Table]**

| Ingredients | Content (Unit; wt%) |
|---|---|
| Purified Water | Residue |
| Glycerin | 8.0 |
| Butylene Glycol | 4.0 |
| Hyaluronic Acid Extract | 5.0 |
| Beta Glucan | 7.0 |
| Carbomer | 0.1 |
| 4',6,7-trihydroxyisoflavone or 3',4',7-trihydroxyisoflavone | 0.05 |
| Caprylic/Capric Triglyceride | 8.0 |
| Squalane | 5.0 |
| Cetearyl Glucoside | 1.5 |
| Sorbitan Stearate | 0.4 |
| Cetearyl Alcohol | 1.0 |
| Triethanol Amine | 0.1 |

### Formulation Example 2: Nourishing cream

According to the composition shown in Table 2 below, a nourishing cream was prepared using a conventional method.

### Table 2

### [Table 2]

**[Table]**

| Ingredients | Content (Unit; wt%) |
|---|---|
| Purified Water | Residue |
| Glycerin | 3.0 |
| Butylene Glycol | 3.0 |
| Liquid Paraffin | 7.0 |
| Beta Glucan | 7.0 |
| Carbomer | 0.1 |
| 4',6,7-trihydroxyisoflavone or 3',4',7-trihydroxyisoflavone | 3.0 |
| Caprylic/Capric Triglyceride | 3.0 |
| Squalane | 5.0 |
| Cetearyl Glucoside | 1.5 |
| Sorbitan Stearate | 0.4 |
| Polysorbate 60 | 1.2 |
| Triethanol Amine | 0.1 |

### Formulation Example 3: Massage cream

According to the composition shown in Table 3 below, a massage cream was prepared using a conventional method.

### Table 3

### [Table 3]

**[Table]**

| Ingredients | Content (Unit; wt%) |
|---|---|
| Purified Water | Residue |
| Glycerin | 8.0 |
| Butylene Glycol | 4.0 |
| Liquid Paraffin | 45.0 |
| Beta Glucan | 7.0 |
| Carbomer | 0.1 |
| 4',6,7-trihydroxyisoflavone or 3',4',7-trihydroxyisoflavone | 1.0 |
| Caprylic/Capric Triglyceride | 3.0 |
| Bees Wax | 4.0 |
| Cetearyl Glucoside | 1.5 |
| Sorbitan Sesquioleate | 0.9 |
| Vaseline | 3.0 |
| Paraffin | 1.5 |

### Formulation Example 4: Pack

According to the composition shown in Table 4 below, a pack was prepared using a conventional method.

### Table 4

### [Table 4]

**[Table]**

| Ingredients | Content (Unit; wt%) |
|---|---|
| Purified Water | Residue |
| Glycerin | 4.0 |
| Polyvinyl Alcohol | 15.0 |
| Hyaluronic Acid Extract | 5.0 |
| Beta Glucan | 7.0 |
| Allantoin | 0.1 |
| 4',6,7-trihydroxyisoflavone or 3',4',7-trihydroxyisoflavone | 0.5 |
| Nonylphenyl Ether | 0.4 |
| Polysorbate 60 | 1.2 |
| Ethanol | 6.0 |

### Formulation Example 5: Ointment

According to the composition shown in Table 5 below, an ointment was prepared using a conventional method.

### Table 5

### [Table 5]

**[Table]**

| Ingredients | Content (Unit; wt%) |
|---|---|
| Purified Water | Residue |
| Glycerin | 8.0 |
| Butylene Glycol | 4.0 |
| Liquid Paraffin | 15.0 |
| Beta Glucan | 7.0 |
| Carbomer | 0.1 |
| 4',6,7-trihydroxyisoflavone or 3',4',7-trihydroxyisoflavone | 1.0 |
| Caprylic/Capric Triglyceride | 3.0 |
| Squalane | 1.0 |
| Cetearyl Glucoside | 1.5 |
| Sorbitan Stearate | 0.4 |
| Cetearyl Alcohol | 1.0 |
| Bees Wax | 4.0 |

## Claims

1. A skin external composition, which contains, as an active ingredient, 4',6,7-trihydroxyisoflavone, 3',4',7-trihydroxyisoflavone or a mixture thereof, which are ortho-dihydroxyisoflavone derivatives for use in inhibiting epidermal hyperproliferation and alleviating inflammatory skin diseases.

2. The skin external composition for use according to Claim 1, wherein the ortho-dihydroxyisoflavone derivatives are contained in an amount of 0.001-30 wt% based on the total weight of the composition.

3. The skin external composition for use according to Claim 1, wherein the composition is formulated into a cosmetic composition selected from among skin softener, astringent lotion, milk lotion, eye cream, nourishing cream, massage cream, cleansing cream, cleansing foam, cleansing water, powder, essence and pack.

4. The skin external composition for use according to Claim 1, wherein the composition is formulated into a pharmaceutical composition selected from among ointment, gel, cream, patch and spray.

## Patentansprüche

1. Eine Zusammensetzung zur äußeren Anwendung auf der Haut, die als wirksamen Inhaltsstoff 4',6,7-Trihydroxyisoflavon, 3',4',7-Trihydroxyisoflavon oder eine Mischung daraus enthält, die Ortho-Dihydroxyisoflavonderivate zur Verwendung beim Hemmen einer epidermalen Hyperproliferation und Mildern entzündlicher Hautkrankheiten sind.

2. Die Zusammensetzung zur äußeren Anwendung auf der Haut zur Verwendung nach Anspruch 1, wobei die Ortho-Dihydroxyisoflavonderivate in einer Menge von 0,001-30 Gewichtsprozent auf der Basis des Gesamtgewichts der Zusammensetzung enthalten sind.

3. Die Zusammensetzung zur äußeren Anwendung auf der Haut zur Verwendung nach Anspruch 1, wobei die Zusammensetzung in eine kosmetische Zusammensetzung formuliert ist, die aus einem Hautweichmacher, einer adstringierenden Lotion, einer Milchlotion, einer Augencreme, einer Nährcreme, einer Massagecreme, einer Reinigungscreme, einem Reinigungsschaum, einem Reinigungswasser, einem Puder, einer Essenz und einem Umschlag ausgewählt ist.

4. Die Zusammensetzung zur äußeren Anwendung auf der Haut zur Verwendung nach Anspruch 1, wobei die Zusammensetzung in eine pharmazeutische Zusammensetzung formuliert ist, die aus einer Salbe, einem Gel, einer Creme, einem Pflaster und einem Spray ausgewählt ist.

## Revendications

1. Composition externe pour la peau, qui contient, en tant que substance active, de la 4',6,7-trihydroxyisoflavone, de la 3',4',7-trihydroxyisoflavone ou un mélange de celles-ci, qui sont des dérivés d'ortho-dihydroxyisoflavone pour utilisation dans l'inhibition de l'hyperprolifération épidermique et l'atténuation des affections cutanées inflammatoires.

2. Composition externe pour la peau pour utilisation selon la revendication 1, dans laquelle les dérivés d'ortho-dihydroxyisoflavone sont contenus en une quantité de 0,001 à 30 % en poids sur la base du poids total de la composition.

3. Composition externe pour la peau pour utilisation selon la revendication 1, dans laquelle la composition est préparée sous la forme d'une composition cosmétique choisie parmi un adoucissant pour la peau, une lotion astringente, un lait, une crème pour les yeux, une crème nourrissante, une crème de massage, une crème nettoyante, une mousse nettoyante, une eau nettoyante, une poudre, une essence et une compresse.

4. Composition externe pour la peau pour utilisation selon la revendication 1, dans laquelle la composition est formulée dans une composition pharmaceutique choisie parmi un onguent, un gel, une crème, un timbre et une pulvérisation.
